# EUROPEAN PATENT SPECIFICATION

(11) **EP 1 574 193 B1**
(45) Date of publication and mention of the grant of the patent: **25.04.2012**
(21) Application number: 03751312.4
(22) Date of filing: 02.10.2003
(51) Int. Cl.: A61F 13/494

(54) **DISPOSABLE WEARING ARTICLE**
TRAGBARER EINWEGARTIKEL
ARTICLE JETABLE DESTINE A ETRE PORTE

(30) Priority: 23.10.2002 JP 2002307888
(43) Date of publication of application: 14.09.2005
(73) Proprietor: Zuiko Corporation, Osaka 566-0045 (JP)
(72) Inventor: KURATA, Shuhei, c/o Zuiko Corporation, Settsu-shi, Osaka 566-0045 (JP); UMEBAYASHI, Toyoshi, c/o Zuiko Corporation, Settsu-shi, Osaka 566-0045 (JP)
(74) Representative: Daniels, Jeffrey Nicholas
(86) International application number: PCT/JP2003/012629
(87) International publication number: WO 2004/037145

(56) References cited:
- EP-A- 1 201 212
- EP-A- 1 285 644
- WO-A-01/74280
- JP-A- 2000 093 462
- US-B1- 6 179 820

## Description

### TECHNICAL FIELD

The present invention relates to a disposable wearing article.

### BACKGROUND ART

WO01/74280 A discloses an absorbent article with elastic Y-shaped means. US-B1-6179820 discloses an absorbent article. EP-A-1285644, which is citable under Article 54(3) EPC, discloses a pants-type disposable diaper.

Heretofore, there have been known various disposable pant-type body-fluid-absorbent wearing articles, such as an infant pant-type diaper or training pant, an adult diaper, an incontinence pant and a ladies' menstrual sanitary pant. In such disposable wearing articles, an absorbent body is attached on a skin-side surface of a cover sheet, as disclosed in Japanese Patent Laid-Open Publication No. 09-56747.

Among the above disposable wearing articles, the menstrual sanitary pant particularly has a strong need for assuring a close fit of the entire absorbent body to the entire crotch region of a wearer.

Further, there remains a need for providing a disposable wearing article having a good appearance during use.

In view of the above needs, it is an object of the present invention to provide a disposable wearing article having enhanced fit of the entire absorbent body to the entire crotch region of a wearer.

### DISCLOSURE OF INVENTION

The present invention provides a disposable wearing article comprising an absorbent body, and a primary elastic member attached to a back portion of said absorbent body to extend a given length longitudinally and approximately linearly in an approximately laterally central zone of said back portion, wherein said primary elastic member has a pair of right and left portions forming a linear convergent portion in which the right and left portions extend a given length (H2) in convergent relation to one another in an approximately laterally central zone of the absorbent body while keeping such a constant distance (H1) therebetween that the action of a contractile force in the linear convergent portion of the primary elastic member allows a central zone of a skin-side surface of the absorbent body between said right and left portions to be formed as a raised portion in the given length, and extends forwardly from the front end of said linear convergent portion in a forwardly divergent pattern and/or rearwardly from the rear end of said linear convergent portion in a rearwardly divergent pattern, wherein the distance H1 is in the range of 5 to 50 mm and the length H2 is in the range of 100 to 350 mm.

In the above disposable wearing article, the approximately linear portion of the primary elastic member is attached to the approximately laterally central zone of the back portion of the absorbent body. Thus, the action of a contractile force of the primary elastic member allows a central zone of a skin-side surface of the absorbent body to be raised in a given length so as to facilitate a close fit of the absorbent body to the central area of a crotch region of a wearer.

Further, the primary elastic member has a linear convergent portion and extend forwardly from the front end of the linear convergent portion in a forwardly divergent pattern. The action of a contractile force in the forwardly-divergent portion of the primary elastic member allows a front zone of the skin-side surface of the absorbent body to be raised in a forwardly divergent pattern so as to facilitate a close fit of the absorbent body to the front area of the crotch region.

The primary member also extends rearwardly from the rear end of the linear convergent portion in a rearwardly divergent pattern. The action of a contractile force in the rearwardly-divergent portion of the primary elastic member allows a rear zone of the skin-side surface of the absorbent body to be raised in a rearwardly divergent pattern so as to facilitate a close fit of the absorbent body to the rear area of the crotch region (valley area in a hip region of the wearer).

The above disposable wearing article may further include a secondary elastic member attached to each of opposite lateral edges of the back portion of the absorbent body to extend a given length approximately linearly. In this case, the action of respective contractile forces of the primary and secondary elastic members allows the central zone of the skin-side surface of the absorbent body to be raised in a given length and at an increased height.

Preferably, the absorbent body includes a pair of backsheets superimposed on one another, a topsheet, and an absorbent core, wherein the primary elastic member and/or the secondary elastic member are attached between the backsheets.

Preferably, the above disposable wearing article further includes a risable flap disposed along each of opposite lateral edges of the absorbent body.

Preferably, the above disposable wearing article further includes a cover sheet having an abdominal-side front zone, a dorsal-side rear zone, and a crotch zone located between the front and rear zones and formed with a leg opening along each of opposite lateral edges thereof.

The above absorbent body may be replaceably attached to a cover sheet having no absorbent body (pant, diaper, etc.), or may be replaceably attached to a disposable pant-type absorbent body in a superimposed manner.

The present description also provides a disposable wearing article comprising a cover sheet, an absorbent body attached on a skin-side surface of the cover sheet, and a primary elastic member attached to the cover sheet to extend a given length longitudinally and approximately linearly in an approximately laterally central zone of the cover sheet.

In the above disposable wearing article, the approximately linear portion of the primary elastic member is attached to the approximately central zone of the disposable pant-type cover sheet. Thus, the action of a contractile force of the primary elastic member allows a central zone of the skin-side surface of the cover sheet to be raised in a given length. This makes it possible to raise a central zone of a skin-side surface of the absorbent body in a given length so as to facilitate a close fit of the absorbent body to the central area of a crotch region of a wearer.

Further, the primary elastic member may have a linear convergent portion and extend forwardly from the front end of the linear convergent portion in a forwardly divergent pattern. In this case, the action of a contractile force in the forwardly-divergent portion of the primary elastic member allows a front zone of the skin-side surface of the cover sheet to be raised in a forwardly divergent pattern. This makes it possible to raise a front zone of the skin-side surface of the absorbent body in a forwardly divergent pattern so as to facilitate a close fit to the front area of the crotch region.

The primary elastic member may also extend rearwardly from the rear end of the linear convergent portion in a rearwardly divergent pattern. In this case, the action of a contractile force in the rearwardly-divergent portion of the primary elastic member allows a rear zone of the skin-side surface of the cover sheet to be raised in a rearwardly divergent pattern. This makes it possible to raise a rear zone of the skin-side surface of the absorbent body in a rearwardly divergent pattern so as to facilitate a close fit to the rear area of the crotch region (valley area in a hip region of the wearer).

Furthermore, the present description provides a method of producing a disposable wearing article, which comprises the steps of: cutting a cover sheet into two pieces in the longitudinal direction thereof, and spacing the two cover-sheet pieces from one another; attaching an absorbent body to the respective spaced cover-sheet pieces in such a manner as to bridge therebetween; and forming a leg opening.

The disposable wearing article produced through this method can have a pant-like configuration during use to provide an improved appearance.

### BRIEF DESCRIPTION OF DRAWINGS

FIG. 1 is an exploded perspective view of an absorbent body.
FIG. 2A is a perspective view of an absorbent body provided with a primary elastic member.
FIG. 2B is a top plan view of the absorbent body in FIG. 2A.
FIG. 2C is a perspective view of an absorbent body provided with primary and secondary elastic members.
FIG 2D is a top plan view of the absorbent body in FIG. 2C.
FIG. 3A is a perspective view of an absorbent body, wherein the absorbent body is horizontally stretched.
FIG 3B is a perspective view of the absorbent body in FIG. 3A, wherein the absorbent body is three-dimensionally deformed.
FIG 4 is an exploded perspective view of a disposable pant.
FIGS. 5A to 5D are top plan views showing various examples of the attachment of a leg elastic member.
FIG. 6 is an exploded perspective view of a disposable pant.
FIGS. 7A to 7C are top plan views showing various examples of the attachment of a leg elastic member.
FIGS. 8A to 8C are top plan views showing various examples of the attachment of a leg elastic member and a body-fit elastic member.
FIGS. 9A to 9D are top plan views showing various examples of respective shapes of an abdominal-side front zone and a dorsal-side rear zone, and the attachment of a body-fit elastic member and a leg elastic member.
FIG. 9E is an explanatory top plan view showing an S-cutting.
FIGS. 10A to 10C show the disposable pant in FIG. 4, wherein FIGS. 10A, 10B and 10C are a perspective front view, a perspective back view and a fragmentally sectional view of the disposable pant, respectively.
FIGS. 11A to 11C show the disposable pant in FIG. 6, wherein FIGS. 11A, 11B and 11C are a perspective front view, a perspective back view and a fragmentally sectional view of the disposable pant, respectively. ' FIG. 12 is an exploded perspective view of a disposable pant as a first example.
FIG. 13 is an exploded perspective view of a disposable pant as a second example.
FIGS. 14A to 14C show the disposable pant in FIG. 12, wherein FIGS. 14A, 14B and 14C are a perspective front view, a perspective back view and a fragmentally sectional view of the disposable pant, respectively.
FIGS. 15A to 15C show the disposable pant in FIG. 13, wherein FIGS. 15A, 15B and 15C are a perspective front view, a perspective back view and a fragmentally sectional view of the disposable pant, respectively.

### BEST MODE FOR CARRYING OUT THE INVENTION

With reference to the drawings, an embodiment of the present invention will now be described in detail.

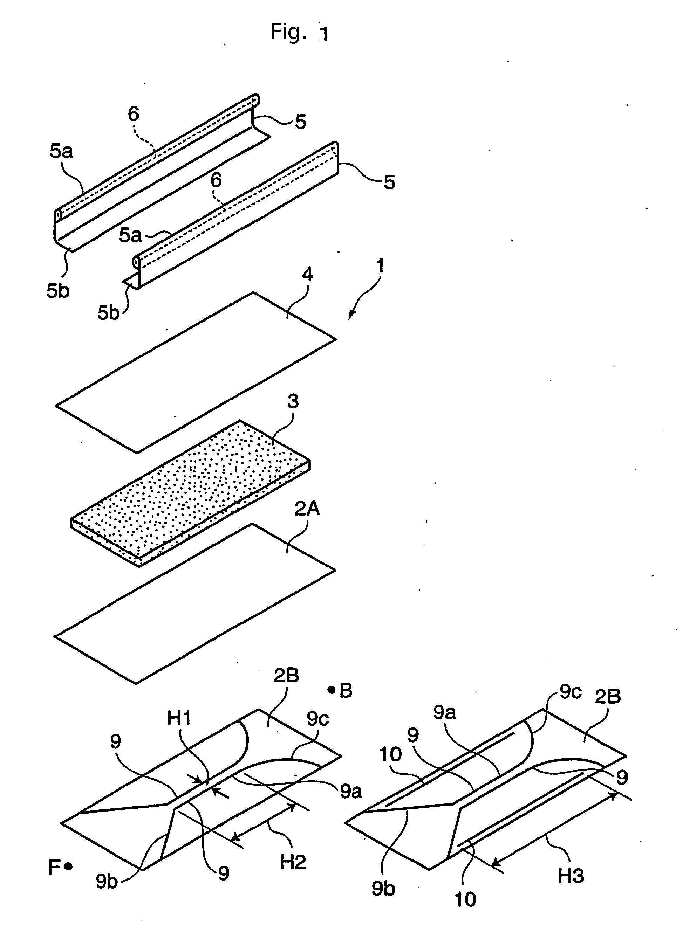

As shown in FIGS. 1 to 3B, an absorbent body 1 serving as a disposable wearing article fundamentally comprises a pair of backsheets 2 (A, B), an absorbent core 3, a topsheet 4, and a pair of right and left risable flaps 5.

At least one of the backsheets 2 (A, B) is made of a liquid-impervious material. A synthetic resin film or a water-shedding nonwoven may be suitably used as the liquid-impermeable material, and a nonwoven may be suitably used as a liquid-pervious martial therefor. An after-mentioned primary elastic member 9 is attached between the two backsheets 2 (A, B) to be superimposed on one another. Instead of making the backsheets 2 (A, B) of the absorbent body 1 with a liquid-impervious material, an additional sheet made of a liquid-impervious material may be disposed under the bottom of the absorbent core 3.

The absorbent core 3 is made of a natural pulp fiber, a synthetic resin fiber and/or a superabsorbent resin material.

The topsheet 4 is made of a liquid-pervious nonwoven.

The pair of right and left risable flaps 5 are made of a synthetic resin nonwoven having water-shedding properties or waterproofing properties. Each of the risable flaps 5 includes a flap elastic member 6 bonded in its stretched state to a free end 5a thereof to extend the longitudinal direction of the absorbent body.

The elastic member 6 may be made of polyurethane, natural rubber or film, and may be formed in a thread-like shape or a ribbon-like shape. The number of the elastic member 6 is not limited to one, but a plurality of elastic members 6 may be used. The same goes for after-mentioned various elastic members, such as primary and secondary elastic members 9, 10.

The absorbent body 1 is formed by bonding the absorbent core 3 onto the upper backsheet 2A, bonding the topsheet 4 onto the upper backsheet 2A in such a manner as to wrap around the absorbent core 3, and bonding the anchor end 5b of each of the risable flaps 5 to a corresponding one of opposite lateral edges of the obtained assembly.

A primary elastic member 9 is attached in its stretched state between the two backsheets 2 (A, B) superimposed on each other.

The primary elastic member 9 has of a pair of right and left portions extending a given length H2 in convergent relation to one another while keeping a constant distance H1 therebetween, in an approximately laterally central zone of the absorbent body 1. The distance H1 is set in the range of 5 to 50 mm, and the length H2 is set in the range of 100 to 350 mm.

As shown in FIG. 1, the primary elastic member 9 extends forwardly F from the front end of the convergent portion 9a in a forwardly divergent pattern to form a forwardly-divergent portion 9b. The primary elastic member 9 also extends rearwardly B from the rear end of the convergent portion 9a in a rearwardly divergent pattern to form a rearwardly-divergent portion 9c. Each of the forwardly-divergent portion 9b and the rearwardly-divergent portion 9c may be formed in an approximately V shape or may be formed in a curved shape.

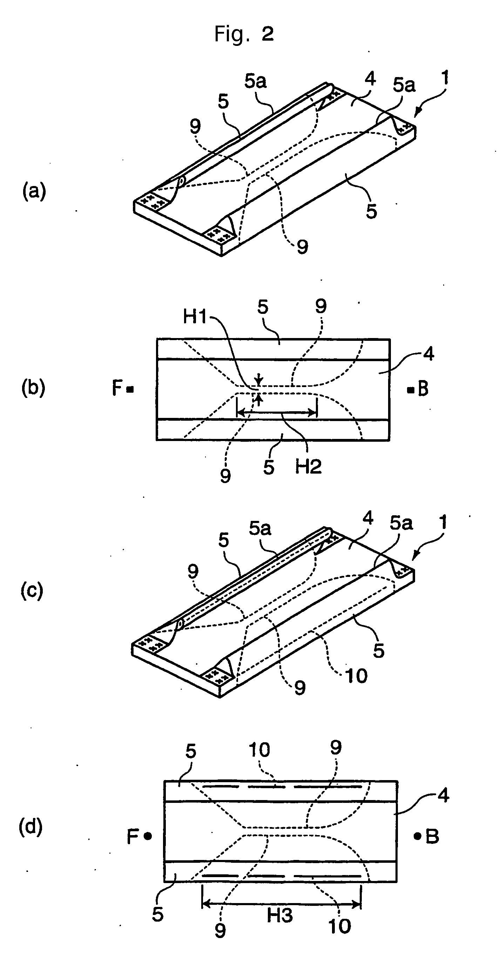

As shown in the lower right portion of FIG. 1 and in FIGS. 2C and 2D, a pair of right and left secondary elastic members 10 may be attached, respective, to opposite lateral edges of the absorbent body to extend a given length H3 approximately linearly in the stretched state of the absorbent body 1.

While the length H3 is preferably set to prevent the secondary elastic members 10 from intersecting with the forwardly-divergent portion 9b and the rearwardly-divergent portion 9c of the primary elastic member 9, it may be set to allow the intersect therebetween.

According to the absorbent body 1 constructed as above, the approximately linear portion of the primary elastic member 9 is attached to the approximately central zone of the back portion of the absorbent body 1, and formed as the convergent portion 9a. Thus, the action of a contractile force in the convergent portion 9a of the primary elastic member 9 allows a central zone of a skin-side surface of the absorbent body 1 to be formed as a raised portion in a given length H2 so as to so as to facilitate a close fit of the absorbent body 1 to the central area of a crotch region of a wearer.

The primary elastic member 9 further extends rearwardly from the rear end of the linear portion in a rearwardly divergent pattern to form the rearwardly-divergent portion 9c. The action of a contractile force in the rearwardly-divergent portion 9c of the primary elastic member 9 allows a rear zone of the skin-side surface of the absorbent body 1 to be raised in a rearwardly divergent pattern so as to facilitate a close fit to the rear area of the crotch region (valley area in a hip region of the wearer).

Furthermore, the primary elastic member 9 extends forwardly from the front end of the linear convergent portion 9a in a forwardly divergent pattern. The action of a contractile force in the forwardly-divergent portion 9b of the primary elastic member 9 allows a front zone of the skin-side surface of the absorbent body 1 to be raised in a forwardly divergent pattern so as to facilitate a close fit to the front area of the crotch region. This provides enhanced fit of the entire absorbent body 1 to the entire crotch region, whereby the absorption speed of body fluid can be increased to completely eliminate the risk of side leakage of the body fluid.

When the secondary elastic member 10 is additionally attached to each of the opposite lateral edges of the back portion of the absorbent body 1 to extend the given length H3 approximately linearly, the action of respective contractile forces of the primary and secondary elastic members 9, 10 allows the central zone of the skin-side surface of the absorbent body 1 to be raised in the given length H2 and at an increased height so as to provide further enhanced fit to the central area of the crotch region.

While it is optimal that the primary elastic member 9 comprises a combination of the convergent portion 9a, the forwardly-divergent portion 9b and the rearwardly-divergent portion 9c, the primary elastic member 9 may have only the convergent portion 9a, or may have a combination of the convergent portion 9a and the forwardly-divergent portion 9b, or may have a combination of the convergent portion 9a and the rearwardly-divergent portion 9c.

Further, while the primary and secondary elastic members 9, 10 may be attached between the absorbent core 3 and a single backsheet 2 of the absorbent body 1, it is desirable to attach the primary and secondary elastic members 9, 10 between the two backsheets 2 (A, B) superimposed on one another so as to allow a line production of the absorbent body 1 to be facilitated.

In addition, the risable flaps 5 attached to the opposite lateral edges of the absorbent body 1 makes it possible to reliably prevent side leakage from the sides of the absorbent body 1.

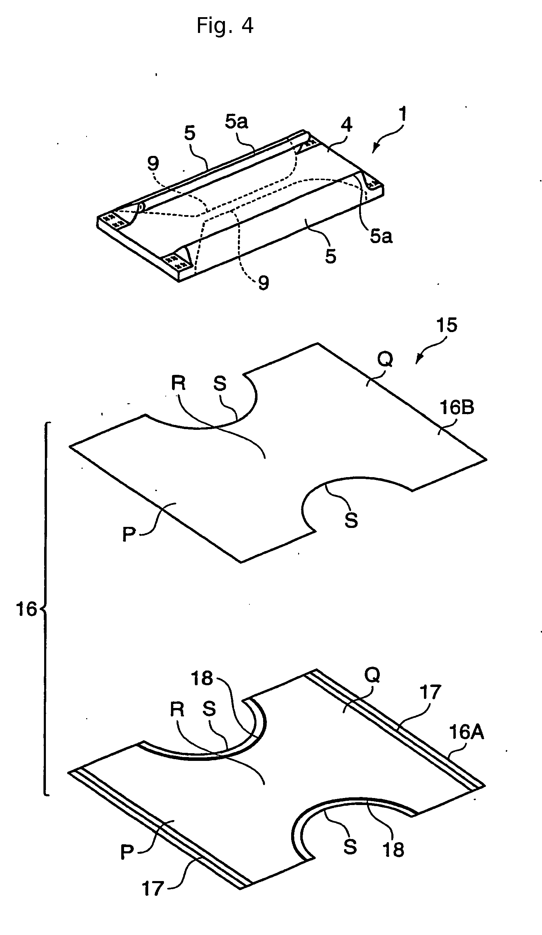

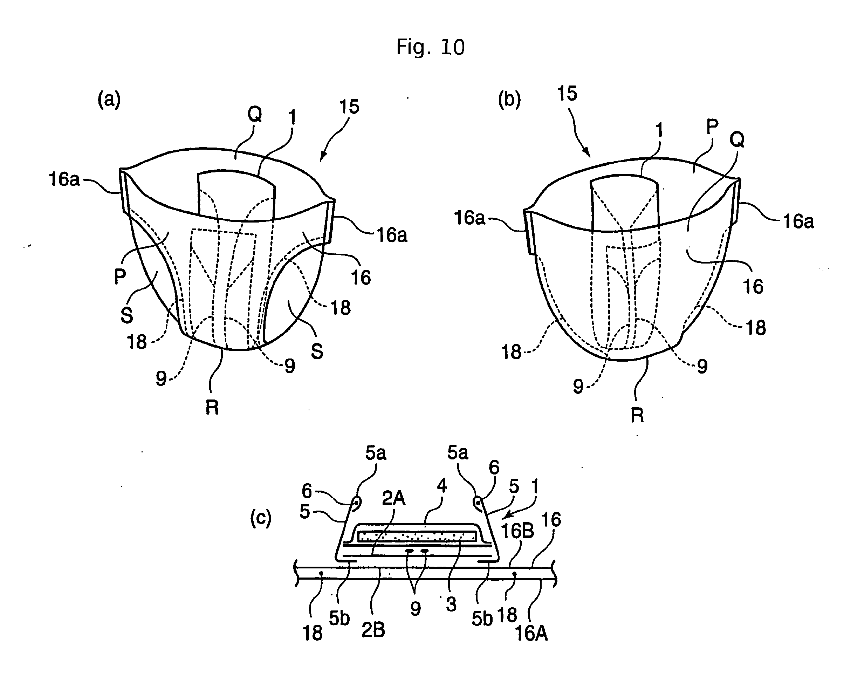

The absorbent body 1 may be replaceably attached to a cover sheet having no absorbent body 1 (pant, diaper, etc.) or may be replaceably attached to a disposable pant-type absorbent body in a superimposed manner. The following description will be made in connection with an example where the technique of raising a portion of the absorbent body 1 is applied to a disposable pant and a menstrual sanitary article (hereinafter referred to collectively as "pant" for short), with reference to FIGS. 4 to 11C.

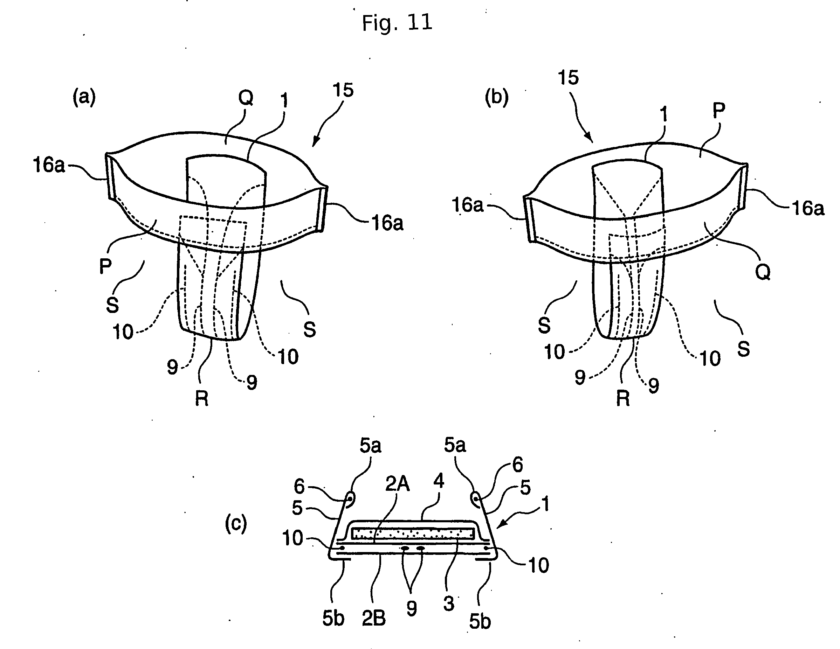

As shown in FIGS. 4 to 11C, a cover sheet 16 of a pant 15 is made, for example, of a breathable nonwoven. The cover sheet 16 comprises an outer sheet 16A and a skin-side sheet 16B each formed to have a contour of the pant. Each of the outer sheet 16A and the skin-side sheet 16B is formed with a leg opening S along each of opposite lateral edges of a crotch zone R located between an abdominal-side front zone P and a dorsal-side rear zone Q of the pant 15. Preferably, the outer sheet 16A and/or the skin-side sheet 16B are made of a breathable nonwoven.

A waist elastic member 17 is attached in its stretched state between the outer sheet 16A and the skin-side sheet 16B to be bonded to one another in a superimposed manner, along each of front and rear ends thereof, and a leg elastic member 18 is attached in its stretched state around each of the leg openings S.

The aforementioned absorbent body 1 is bonded onto the crotch zone R of the skin-side sheet 16B.

Then, the pant 15 is formed by bending the cover sheet 16 along the crotch zone R while locating the skin-side sheet 16B inside, and joining respective opposed lateral edges 16a of the cover sheet 16 together.

As in the description about the absorbent body 1, the pant 15 formed as above also provides enhanced fit of the entire absorbent body 1 to the entire crotch region of a wearer, so that the absorption speed of body fluid can be increased to completely eliminate the risk of side leakage of the body fluid.

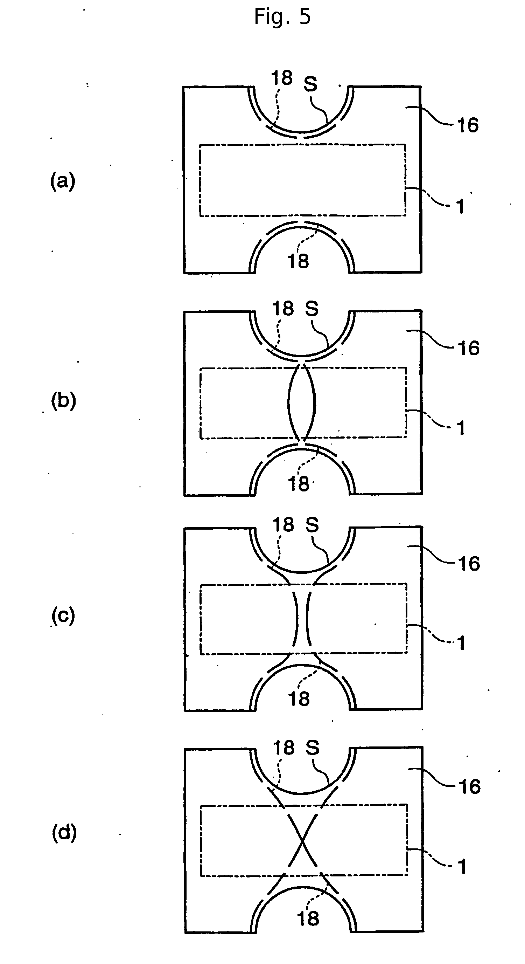

FIGS. 5A to 5D show various examples of the attachment of the leg elastic member 8, wherein FIG. 5A shows one example where a leg elastic member is attached to each of the leg openings S in an arc-shaped pattern, FIG. 5B showing another example where leg elastic members are arranged to continuously extend between the right and left leg openings S while intersecting in the crotch zone R in an O-shaped pattern, FIG. 5C showing another example where leg elastic members are arranged to continuously extend between the right and left leg openings S without intersecting in the crotch zone R, and FIG. 5D showing another example where leg elastic members are arranged to continuously extend between the right and left leg openings S while intersecting in the crotch zone R in an X-shaped pattern.

The leg elastic member 18 attached along each of the leg openings S of the cover sheet 16 in this manner makes it possible to prevent sagging in the crotch zone R so as to adequately maintain a close fit of the entire absorbent body 1 to the entire crotch region. This allows the need for providing the risable flaps 5.

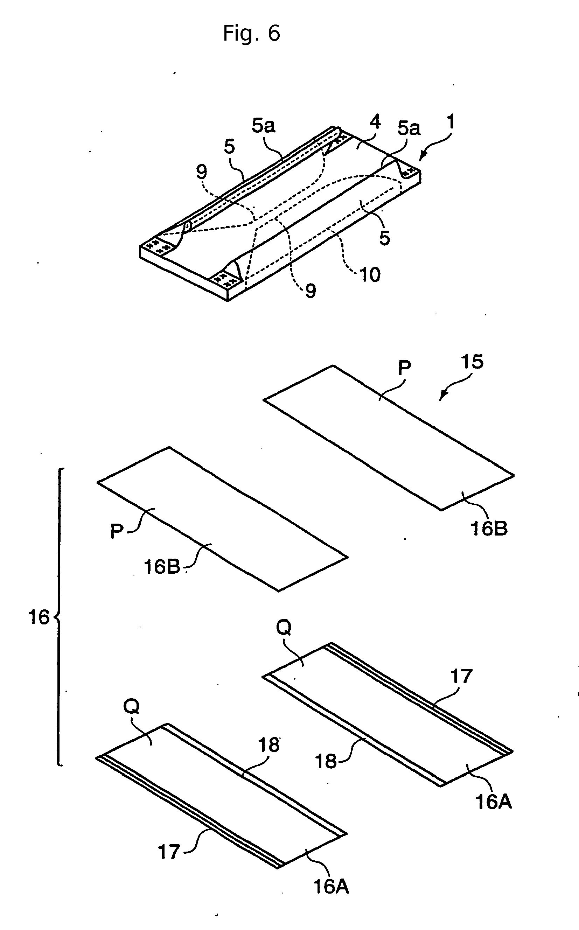

In the embodiment illustrated in FIGS. 4, 5A to 5D, and 10A to 10C, each of the outer sheet 16A and the skin-side sheet 16B of the cover sheet 16 is integrally formed with the leg openings S along the opposite lateral edges of the crotch zone R located between the abdominal-side front zone P and the dorsal-side rear zone Q thereof. Alternatively, another structure may be used as in an embodiment illustrated in FIG. 6, 7A to 7C and 11A to 11C.

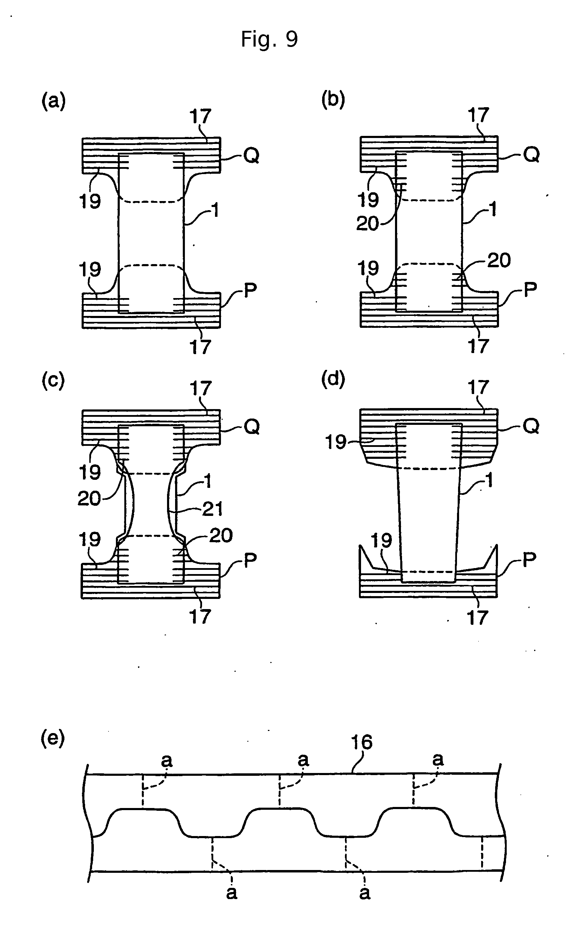

Specifically, a pant 15 in this embodiment is designed such that the abdominal-side front zone P and the dorsal-side rear zone Q are formed, respectively, of a pair of outer sheets 16A and a pair of skin-side sheets 16B, but the crotch zone R is not formed of the cover sheet 16. For example, as shown in FIGS. 9A to 9D, the absorbent body 1 is attached to an elastic belt for allowing the absorbent body 1 to have a close fit to a crotch region of a wearer.

A waist elastic member 17 is attached in its stretched state between each pair of the outer sheet 16A and the skin-side sheet 16B to be bonded to one another in a superimposed manner, along each of front and rear ends thereof, and a leg elastic member 18 is attached in its stretched between each pair of the outer sheet 16A and the skin-side sheet 16B state, along the inward end thereof.

The aforementioned absorbent body 1 is attached to the respective skin-side sheets 16B to bridge between the abdominal-side front zone P and the dorsal-side rear zone Q. That is, the crotch zone R and a pair of leg openings S are defined by the absorbent body 1 itself.

The pant 15 is formed by bending the cover sheet 16 along the crotch zone R of the absorbent body 1 while locating the skin-side sheets 16B inside, and joining respective opposed lateral edges 16a of the cover sheet 16 together.

As in the description about the absorbent body 1, the pant 15 formed as above also provides enhanced fit of the entire absorbent body 1 to the entire crotch region of a wearer, so that the absorption speed of body fluid can be increased to completely eliminate the risk of side leakage of the body fluid.

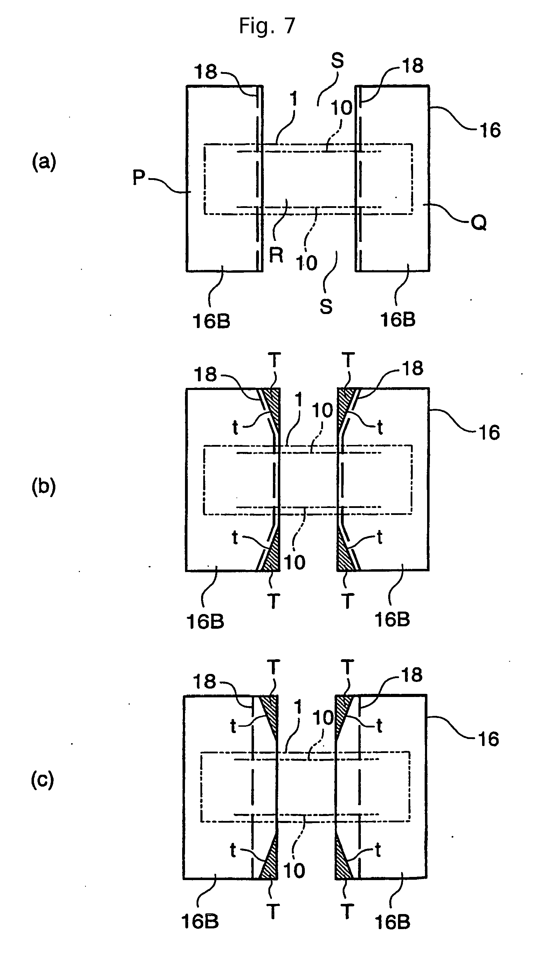

FIGS. 7A to 7C show various examples of the shape of the abdominal-side front zone P and the dorsal-side rear zone Q, and the attachment of the leg elastic member 18, wherein FIG. 7A shows one example where each of the abdominal-side front zone P and the dorsal-side rear zone Q has a rectangular shape, and the leg elastic member 18 is attached to extend linearly in the longitudinal direction, FIG. 7B showing another example where each of the abdominal-side front zone P and the dorsal-side rear zone Q has a trapezoidal shape, and the leg elastic member 18 is attached to extend along the trapezoid, and FIG. 7C showing another example where each of the abdominal-side front zone P and the dorsal-side rear zone Q has a trapezoidal shape, and the leg elastic member 18 is attached to extend linearly in the longitudinal direction. The aforementioned type of absorbent body 1 having the secondary elastic member 10 attached thereto may be advantageously used to serve as an additional leg elastic member 18.

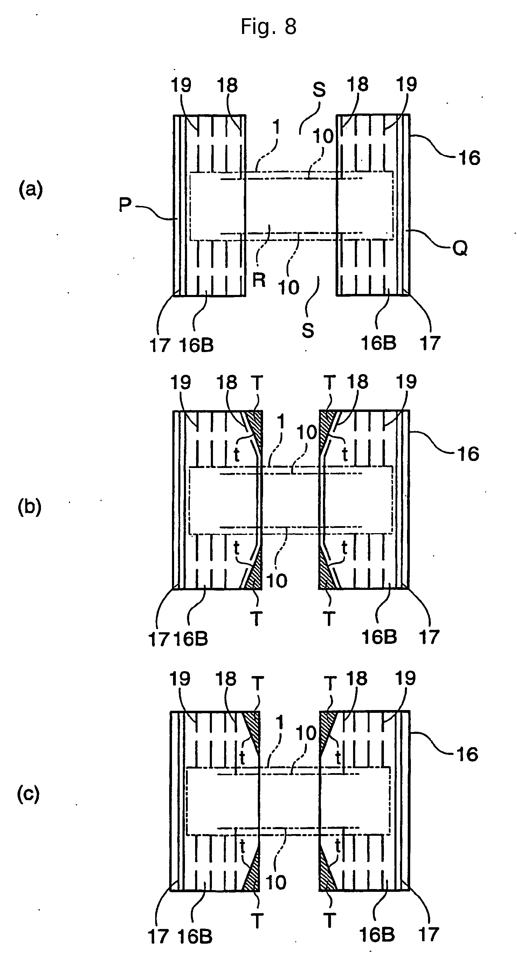

FIGS. 8A to 8C show various examples of the attachment of a body-fit elastic member 19 added to the respective examples of the leg elastic member 18 in FIGS. 7A to 7C. These examples are designed such that the leg elastic member 18 and the body-fit elastic member 19 are attached in such a manner as to be almost excluded from areas located under the absorbent body 1. In these figures, the reference numeral 17 indicates a waist elastic member.

The disposable wearing articles as shown in FIGS. 7A and 8A are produced by cutting a cover sheet into two pieces, spacing the two cover-sheet pieces from one another to form the abdominal-side front zone P and the dorsal-side rear zone Q, mounting the absorbent body in such a manner as to bridge between the abdominal-side front zone P and the dorsal-side rear zone Q, and cutting the abdominal-side front zone P and the dorsal-side rear zone Q at given intervals. The disposable wearing articles as shown in FIGS. 7B, 7C, 8B and 8C are produced by mounting the absorbent body in the above manner, cutting out portions T, and cutting the abdominal-side front zone P and the dorsal-side rear zone Q at given intervals. While the edge t of the portion T is illustrated by a linear line in FIGS. 7B, 7C, 8B and 8C, the edge t may be a curved line. This curved edge t desirably provides further enhanced appearance during use. While FIGS. 7A to 8C show examples of a wearable article incorporating the type of absorbent body having the secondary elastic member 10 attached thereto, any other type of absorbent body may be used.

In the above production process, a part of the absorbent body may be cut out in conjunction with the cutout of the portions T. In this case, the leg opening S can be desirably formed to have a shape capable of providing enhanced fit to the legs of a wearer.

While the above production process has been described in connection with one example where the portions T are cut out after mounting the absorbent body, the portions T may be cut out before mounting the absorbent body, or may be cut out before cutting the cover sheet into two pieces. The step of cutting out the portions T may be performed at any time before the step of cutting the abdominal-side front zone P and the dorsal-side rear zone Q at a position between the adjacent absorbent bodies mounted thereon.

FIGS. 9A to 9D show various examples of respective shapes of the abdominal-side front zone P and the dorsal-side rear zone Q, and the attachment of the body-fit elastic member 19 and the leg elastic member 20.

As shown in FIG. 9E, the abdominal-side front zone P and the dorsal-side rear zone Q are prepared by cutting a cover sheet 16 into two pieces in a longitudinally meandering pattern, cutting each of the cut piece pairs along a plurality cutting lines a, and displacing the phase of each of the cut sub-piece pairs by one-half pitch (so-called "S-cutting").

The abdominal-side front zone P and the dorsal-side rear zone Q prepared through the S-cutting are used in FIGS. 9A to 9C, wherein FIG. 9A shows one example where the body-fit elastic member 19 is attached in such a manner to be excluded from areas located under the absorbent body 1. In these figures, the reference numeral 17 indicates a waist elastic member.

FIG 9B shows another example where the leg elastic member 20 is attached to leg flap areas in the abdominal-side front zone P and the dorsal-side rear zone Q in such a manner to be almost excluded from areas located under the absorbent body 1. FIG. 9C shows another example where the leg elastic member 20 is attached to the leg flap areas in the abdominal-side front zone P and the dorsal-side rear zone Q in such a manner to be almost excluded from areas located under the absorbent body 1, and a central zone of the absorbent body 1 is formed to have a slightly narrowed width to allow a curve-shaped leg gather 21 to be arranged therein.

FIG. 9D shows another example where the leg elastic member 20 is attached to leg flap areas in the abdominal-side front zone P and the dorsal-side rear zone Q prepared through a modified S-cutting (without the phase displacement), in such a manner to be almost excluded from areas located under the absorbent body 1. In this example, the absorbent body 1 formed in a trapezoidal shape (tapered shape).

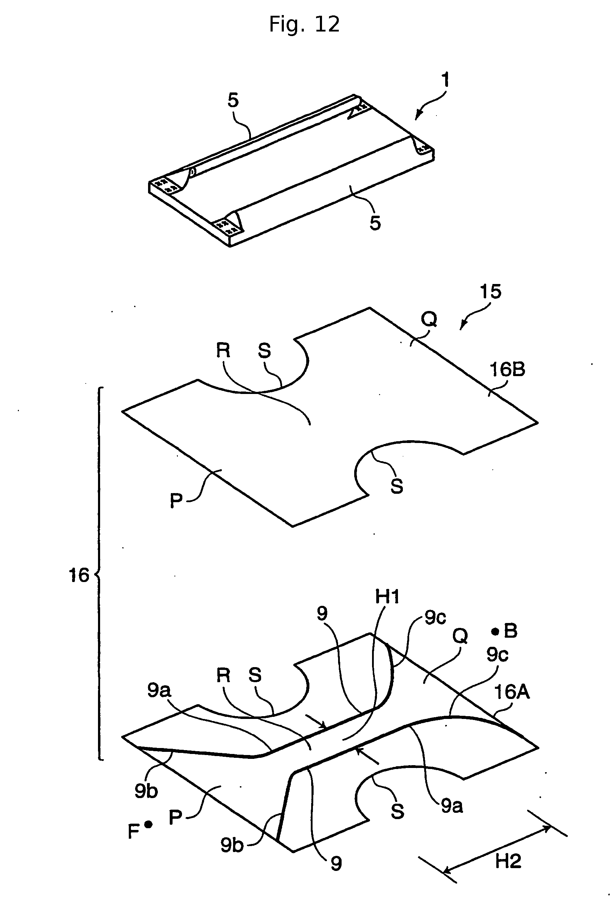

In the above embodiments illustrated in FIG 1 to FIG. 11C, the primary elastic member 9 (and the secondary elastic member 10 according to need) is attached to the absorbent body 1. Alternatively, as in an embodiment illustrated in FIGS. 12 to FIG. 15C, the primary elastic member 9 (secondary elastic member 10) may be attached to a cover sheet 16. While a type of absorbent body 1 having no primary elastic member 9 (secondary elastic member 10) attached thereto is fundamentally used in the embodiment, the type having the primary elastic member 9 (secondary elastic member 10) attached thereto may also be used.

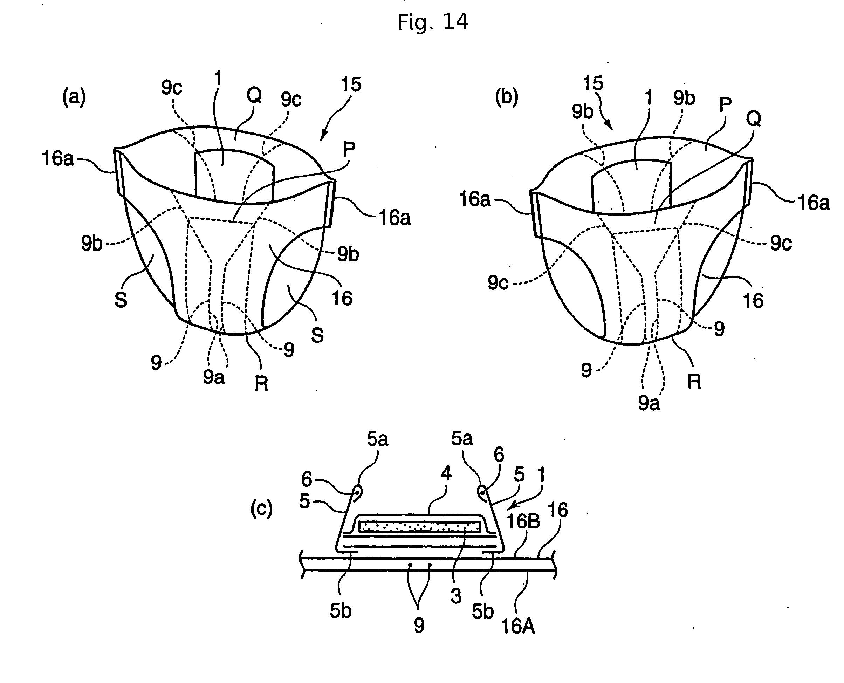

FIGS. 12 and 14A to 14C shows a first example, wherein a cover sheet 16 of a pant 15 is made of a breathable nonwoven, and each of an outer sheet 16A and a skin-side sheet 16B is formed to have a contour of the pant. Further, each of the outer sheet 16A and the skin-side sheet 16B is formed with a leg opening S along each of opposite lateral edges of a crotch zone R located between an abdominal-side front zone P and a dorsal-side rear zone Q of the pant 15.

A primary elastic member 9 is attached in its stretched state between the outer sheet 16A and the skin-side sheet 16B to be bonded to one another in a superimposed manner.

The primary elastic member 9 has of a pair of right and left portions extending a given length H2 in convergent relation to one another while keeping a constant distance H1 therebetween, in an approximately laterally central zone of the absorbent body 1. Preferably, the distance H1 is set in the range of about 5 to 50 mm, and the length H2 is set in the range of about 100 to 350 mm (about 50 to 250 mm for an infant absorbent body).

The primary elastic member 9 illustrated in FIG. 12 extends rearwardly B from the rear end of the convergent portion 9a in a rearwardly divergent pattern to form a rearwardly-divergent portion 9c.

The primary elastic member 9 may further extend forwardly F from the front end of the convergent portion 9a in a forwardly divergent pattern to form a forwardly-divergent portion 9b. Each of the forwardly-divergent portion 9b and the rearwardly-divergent portion 9c may be formed in an approximately V shape or may be formed in a curved shape.

The aforementioned absorbent body 1 is attached onto the crotch zone R of the skin-side sheet 16B.

Then, the pant 15 is formed by bending the cover sheet 16 along the crotch zone R while locating the skin-side sheet 16B inside, and joining respective opposed lateral edges 16a of the cover sheet 16 together.

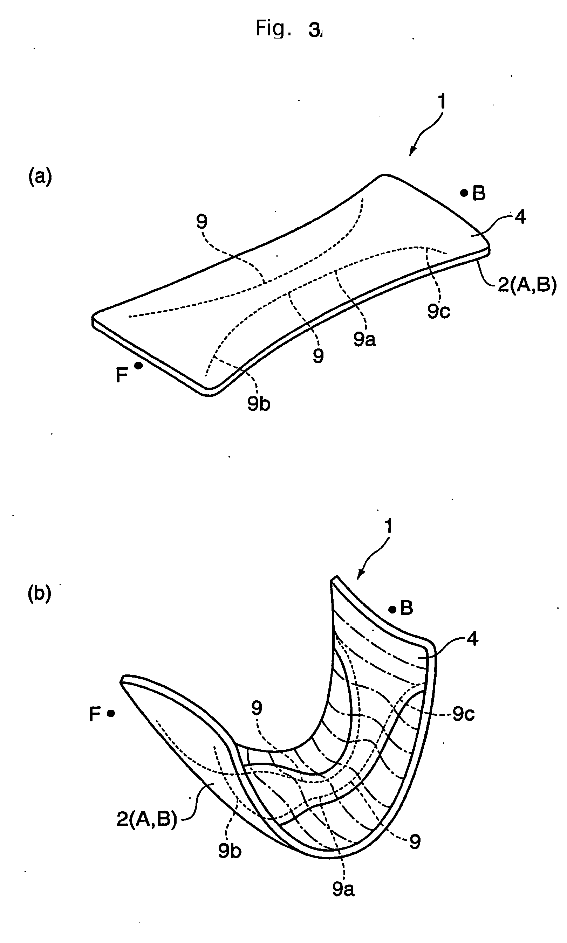

According to the pant 15 as the first example constructed as above, the approximately linear portion of the primary elastic member 9 is attached to the approximately central zone of the cover sheet 16, and formed as the convergent portion 9a. Thus, as with the absorbent body illustrated in FIG. 3, the action of a contractile force in the convergent portion 9a of the primary elastic member 9 allows a central zone of a skin-side surface of the cover sheet 16 to be raised in a given length. This allows a central zone of a skin-side surface of the absorbent body 1 to be formed as a raised portion in a given length H2 so as to facilitate a close fit of the absorbent body 1 to the central area of a crotch region of a wearer.

Further, the primary elastic member 9 extends rearwardly from the rear end of the linear convergent portion 9a in a rearwardly divergent pattern. Thus, the action of a contractile force in the rearwardly-divergent portion 9c of the primary elastic member 9 allows a rear zone of the skin-side surface of the cover sheet 16 to be raised in a rearwardly divergent pattern. This makes it possible to raise a rear zone of the skin-side surface of the absorbent body 1 in a rearwardly divergent pattern so as to facilitate a close fit to the rear area of the crotch region (valley area in a hip region of the wearer).

Furthermore, the primary elastic member 9 extends forwardly from the front end of the linear convergent portion 9a in a forwardly divergent pattern. Thus, the action of a contractile force in the forwardly-divergent portion 9b of the primary elastic member 9 allows a front zone of the skin-side surface of the cover sheet 16 to be raised in a forwardly divergent pattern, and thereby a front zone of the skin-side surface of the absorbent body 1 is raised in a forwardly divergent pattern to facilitate a close fit to the front area of the crotch region. This provides enhanced fit of the entire absorbent body 1 to the entire crotch region, whereby the absorption speed of body fluid can be increased to completely eliminate the risk of side leakage of the body fluid.

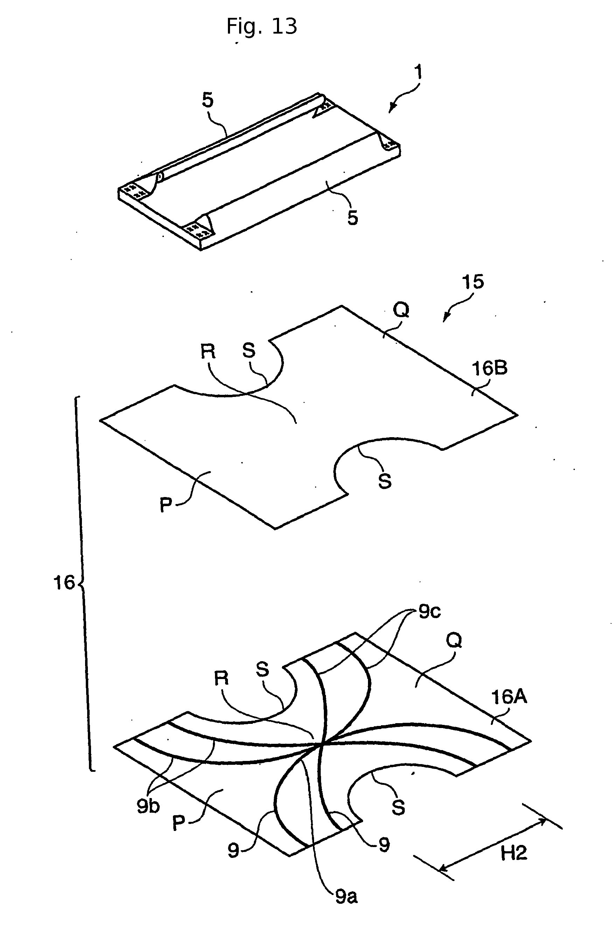

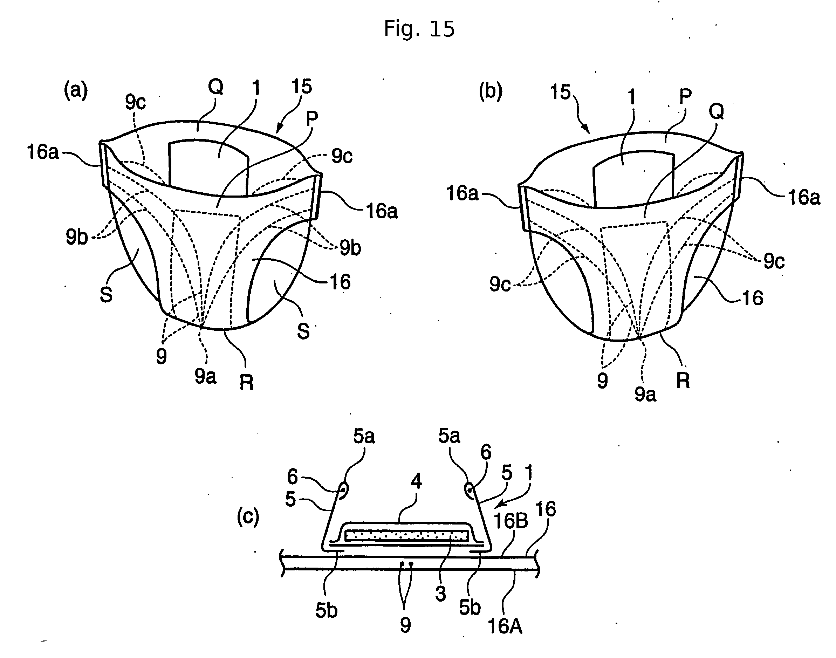

FIGS. 13 and 15A to 15C shows a second example, wherein a cover sheet 16 of a pant 15 is made of a breathable nonwoven, and each of an outer sheet 16A and a skin-side sheet 16B is formed to have a contour of the pant. Further, each of the outer sheet 16A and the skin-side sheet 16B is formed with a leg opening S along each of opposite lateral edges of a crotch zone R located between an abdominal-side front zone P and a dorsal-side rear zone Q of the pant 15.

A primary elastic member 9 is attached in its stretched state between the outer sheet 16A and the skin-side sheet 16B to be bonded to one another in a superimposed manner.

The primary elastic member 9 has of a pair of right and left symmetrical portions intersecting with one another in an approximately laterally central zone of the absorbent body 1 and extending a given length H2 approximately linearly to form a convergent portion 9a. Preferably, the length H2 is set in the range of about 100 to 350 mm (about 50 to 250 mm for an infant absorbent body).

One of the symmetrical portions of the primary elastic member 9 extends extend forwardly F from the front end of the convergent portion 9a in a forwardly divergent pattern to form a forwardly-divergent portion 9b, and the other symmetrical portion extends rearwardly B from the rear end of the convergent portion 9a in a rearwardly divergent pattern to form a rearwardly-divergent portion 9c.

The aforementioned absorbent body 1 is attached onto the crotch zone R of the skin-side sheet 16B.

Then, the pant 15 is formed by bending the cover sheet 16 along the crotch zone R while locating the skin-side sheet 16B inside, and joining respective opposed lateral edges 16a of the cover sheet 16 together.

As with the pant 15 of the first example illustrated in FIGS. 12 and 14A to 14C, the pant 15 of the second example provides enhanced fit of the entire absorbent body 1 to the entire crotch region, and thereby the absorption speed of body fluid can be increased to completely eliminate the risk of side leakage of the body fluid.

In the pant 15 of the second example, a part of the forwardly-divergent portion 9b and the rearwardly-divergent portion 9c of the primary elastic member 9 extending along the abdominal-side front zone P, the dorsal-side rear zone Q and the leg openings S serves as a part of a waist elastic member and a leg elastic member.

### INDUSTRIAL APPLICABILITY

According to the disposable wearing article of the present invention, the approximately linear portion of the primary elastic member is attached to the approximately laterally central zone of the back portion of the absorbent body. Thus, the action of a contractile force of the primary elastic member allows a central zone of a skin-side surface of the absorbent body to be raised in a given length so as to facilitate a close fit of the absorbent body to the central area of a crotch region of a wearer, whereby the absorption speed of body fluid can be increased to eliminate the risk of side leakage of the body fluid.

Further, the primary elastic member has a linear convergent portion and extend forwardly from the front end of the linear convergent portion in a forwardly divergent pattern. The action of a contractile force in the forwardly-divergent portion of the primary elastic member allows a front zone of the skin-side surface of the absorbent body to be raised in a forwardly divergent pattern so as to facilitate a close fit of the absorbent body to the front area of the crotch region.

The primary member also extends rearwardly from the rear end of the linear convergent portion in a rearwardly divergent pattern. The action of a contractile force in the rearwardly-divergent portion of the primary elastic member allows a rear zone of the skin-side surface of the absorbent body to be raised in a rearwardly divergent pattern so as to facilitate a close fit of the absorbent body to the rear area of the crotch region (valley area in a hip region of the wearer). In particular, a combination of the above structures provides enhanced fit of the entire absorbent body to the entire crotch region, so that the absorption speed of body fluid can be further increased to completely eliminate the risk of side leakage of the body fluid.

A secondary elastic member may be additionally attached to each of opposite lateral edges of the back portion of the absorbent body to extend a given length approximately linearly. In this case, the action of respective contractile forces of the primary and secondary elastic members allows the central zone of the skin-side surface of the absorbent body to be raised in a given length and at an increased height so as to provide further enhanced fit to the central area of the crotch region.

The absorbent body may include a pair of backsheets superimposed on one another, a topsheet, and an absorbent core, wherein the primary elastic member and/or the secondary elastic member are attached between the backsheets. This allows a lime production of the absorbent body to be facilitated.

The above disposable wearing article may further include a risable flap disposed along each of opposite lateral edges of the absorbent body to reliable prevent side leakage from the sides of the absorbent body.

The above disposable wearing article may further include a cover sheet formed with a leg opening in each of opposite lateral edges of a crotch zone located between an abdominal-side front zone and a dorsal-side rear zone, and the absorbent body may be the crotch zone of a skin-side surface of the cover sheet, to obtain a disposable pant-type wearing article.

According to another disposable wearing article of the present description, an approximately linear portion of a primary elastic member is attached to an approximately central zone of a disposable pant-type cover sheet. Thus, the action of a contractile force of the primary elastic member allows a central zone of the skin-side surface of the cover sheet to be raised in a given length, and thereby a central zone of a skin-side surface of the absorbent body is raised in a given length so as to facilitate a close fit of the absorbent body to the central area of a crotch region of a wearer, whereby the absorption speed of body fluid can be increased to eliminate the risk of side leakage of the body fluid

Further, the primary elastic member may have a linear convergent portion and extend forwardly from the front end of the linear convergent portion in a forwardly divergent pattern. In this case, the action of a contractile force in the forwardly-divergent portion of the primary elastic member allows a front zone of the skin-side surface of the cover sheet to be raised in a forwardly divergent pattern. This makes it possible to raise a front zone of the skin-side surface of the absorbent body in a forwardly divergent pattern so as to facilitate a close fit to the front area of the crotch region.

The primary elastic member may also extend rearwardly from the rear end of the linear convergent portion in a rearwardly divergent pattern. In this case, the action of a contractile force in the rearwardly-divergent portion of the primary elastic member allows a rear zone of the skin-side surface of the cover sheet to be raised in a rearwardly divergent pattern. This makes it possible to raise a rear zone of the skin-side surface of the absorbent body in a rearwardly divergent pattern so as to facilitate a close fit to the rear area of the crotch region (valley area in a hip region of the wearer). In particular, a combination of the above structures provides enhanced fit of the entire absorbent body to the entire crotch region, so that the absorption speed of body fluid can be further increased to completely eliminate the risk of side leakage of the body fluid.

## Claims

1. A disposable wearing article comprising an absorbent body (1), and a primary elastic member (9) attached to a back portion (2) of said absorbent body to extend a given length longitudinally and approximately linearly in an approximately laterally central zone of said back portion, wherein said primary elastic member has a pair of right and left portions forming a linear convergent portion (9a) in which the right and left portions extend a given length (H2) in convergent relation to one another in an approximately laterally central zone of the absorbent body while keeping such a constant distance (H1) therebetween such that the action of the contractile force in the linear convergent portion of the primary elastic member (9a) allows a central zone of a skin-side surface of the absorbent body between said right and left portions to be formed as a raised portion in the given length (H2) and extends forwardly (F) from the front end of said linear convergent portion in a forwardly divergent pattern and/or rearwardly (B) from the rear end of said linear convergent portion in a rearwardly divergent pattern, wherein the distance (H1) is in the range of 5 to 50 mm and the length (H2) is in the range of 100 to 350 mm.

## Patentansprüche

1. Tragbarer Einwegartikel umfassend einen absorbierenden Körper (1), und ein hauptsächlich elastisches Element (9), das an einem hinteren Bereich (2) des absorbierenden Körpers befestigt ist, um sich in einer bestimmten Länge in Längsrichtung und annähernd linear in einer annähernd seitlich zentralen Zone des hinteren Bereichs zu erstrecken, wobei das hauptsächlich elastische Element ein Paar von rechten und linken Bereichen umfasst, die einen linear zusammenlaufenden Bereich (9a) bilden, bei dem sich die rechten und linken Bereiche über eine bestimmte Länge (H2) in einer zusammenlaufenden Beziehung zueinander in einer annähernd seitlich zentralen Zone des absorbierenden Körpers erstrecken, während sie eine konstante Distanz (H1) dazwischen aufrechterhalten, so dass die Wirkung einer Kontraktionskraft in dem linear zusammenlaufenden Bereich (9a) des hauptsächlich elastischen Elementes es einer zentralen Zone einer Haut-Seiten-Fläche des absorbierenden Körpers zwischen den rechten und linken Bereichen ermöglicht, als ein erhöhter Bereich in der bestimmten Länge (H2) ausgebildet zu werden, und erstreckt sich nach vorne (F) von dem vorderen Ende des linear zusammenlaufenden Bereichs in einer nach vorne auseinanderlaufenden Struktur und/oder nach hinten (B) von dem hinteren Ende des linear zusammenlaufenden Bereichs in einer nach hinten auseinanderlaufenden Struktur, wobei die Distanz (H1) in dem Bereich von 5 bis 50 mm und die Länge (H2) in dem Bereich von 100 bis 350 mm liegt.

## Revendications

1. Article vestimentaire jetable comprenant un corps absorbant (1), et un élément élastique primaire (9) attaché à une partie dorsale (2) dudit corps absorbant pour s'étendre sur une longueur donnée longitudinalement et approximativement linéairement dans une zone centrale approximativement latérale de ladite partie dorsale, où ledit élément élastique primaire a une paire de parties droite et gauche formant une partie convergente linéaire (9a) dans laquelle les parties droite et gauche s'étendent sur une longueur donnée (H2) et convergent l'une vers l'autre dans une zone centrale approximativement latérale du corps absorbant tout en gardant entre elles une distance constante (H1) telle que l'action d'une force de contraction dans la partie linéaire convergente (9a) de l'élément élastique primaire permette à une zone centrale d'une surface côté peau du corps absorbant entre lesdites parties droite et gauche d'être formée comme une partie surélevée dans la longueur donnée (H2), et s'étend vers l'avant (F) à partir de l'extrémité avant de ladite partie convergente linéaire selon un modèle qui diverge vers l'avant et/ou vers l'arrière (B) à partir de l'extrémité arrière de ladite partie convergente linéaire selon un modèle qui diverge vers l'arrière, où la distance (H1) est dans la plage de 5 à 50 mm et la longueur (H2) dans la plage de 100 à 350 mm.
